# EUROPÄISCHE PATENTSCHRIFT

(11) **EP 2 686 035 B1**
(45) Veröffentlichungstag und Bekanntmachung des Hinweises auf die Patenterteilung: **28.01.2015**
(21) Anmeldenummer: 12709529.7
(22) Anmeldetag: 12.03.2012
(51) Int. Cl.: A61M 5/00, B01L 9/06, B01L 9/00, B65D 69/00, B65D 25/10, B65D 1/36

(54) **TRÄGERPLATTE UND TRANSPORT- UND/ODER LAGEREINRICHTUNG FÜR PHARMAZEUTISCHE BEHÄLTNISSE**
CARRIER PLATE AND TRANSPORTING AND/OR STORING DEVICE FOR PHARMACEUTICAL CONTAINERS
PLAQUE SUPPORT ET DISPOSITIF DE TRANSPORT ET/OU DE STOCKAGE POUR DES RECIPIENTS PHARMACEUTIQUES

(30) Priorität: 18.03.2011 DE 102011104300
(43) Veröffentlichungstag der Anmeldung: 22.01.2014
(73) Patentinhaber: SCHOTT Schweiz AG, 9001 St. Gallen (CH)
(72) Erfinder: GERNER, Sebastian, 4056 Basel (CH); FISCHER, Bastian, 9000 St. Gallen (CH)
(74) Vertreter: Sawodny, Michael-Wolfgang
(86) Internationale Anmeldenummer: PCT/EP2012/001095
(87) Internationale Veröffentlichungsnummer: WO 2012/126582

(56) Entgegenhaltungen:
- EP-A1- 2 119 463
- WO-A1-2011/007194
- US-A- 3 643 812
- US-A1- 2009 100 802

## Beschreibung

Die Erfindung betrifft eine Trägerplatte für pharmazeutische Behältnisse, insbesondere Spritzen, Fläschchen oder Karpulen, umfassend einen, eine Mehrzahl von Durchgangsöffnungen zur Aufnahme der pharmazeutischen Behältnisse aufweisenden Aufnahmebereich, einen eine Grundebene definierenden Handhabungsbereich zum Angriff an der Trägerplatte und/oder Lagerung dieser, eine unter Belastung als Druckseite fungierende Oberseite und eine als Zugseite fungierende Unterseite sowie Mittel zur Erhöhung der Steifigkeit der Trägerplatte.

Die Erfindung betrifft ferner eine Transport- und/oder Lagereinrichtung für pharmazeutische Behältnisse.

Zur effektiven Herstellung pharmazeutischer Behältnisse, insbesondere Spritzen, wie vorfüllbare Spritzen, Fläschchen oder Karpulen, werden diese in vordefinierten Anordnungen, so genannten Nestern, vorkonfektioniert, in ihrer Lage zueinander definiert gehalten und so den jeweils erforderlichen Verarbeitungsprozessen gemeinsam und/oder gleichzeitig ausgesetzt. Der Transport und die Lagerung der pharmazeutischen Behältnisse von / zu den und innerhalb der einzelnen, die Verarbeitungsprozesse ausführenden Vorrichtungen sowie die Positionierung innerhalb dieser Vorrichtungen erfolgt ebenfalls in diesen vordefinierten Anordnungen. Dazu werden die pharmazeutischen Behältnisse an einer Aufnahmeeinrichtung in Form einer Trägerplatte in ihrer Lage zueinander und gegenüber der Trägerplatte definiert gehalten und gelagert. Dadurch wird die gleichzeitige Herstellung einer hohen Anzahl derartiger pharmazeutischer Behältnisse in einem Verarbeitungsprozessschritt sichergestellt. Die pharmazeutischen Behältnisse werden im Einzelnen in Aufnahmeöffnungen, insbesondere Durchgangsöffnungen enthaltenden Trägerplatten eingehangen, mit diesen verklemmt oder anderweitig an diesen positioniert, um die Behältnisse beim Transport vor Beschädigungen zu schützen und eine gleichzeitige Weiterverarbeitung der gesamten Behältnisanordnung zu gewährleisten. Insbesondere dienen derartige vordefinierbare Anordnungen mit zentrierter Führung der einzelnen Behältnisse in den jeweiligen Durchgangsöffnungen der Vereinfachung der anordnungsweisen gemeinsamen Weiterverarbeitung in vordefinierten Verfahrensschritten, beispielsweise der Sterilisation der Behältnisse, dem Befüllen der Behältnisse, einem gemeinsamen sicheren Transport zu und von den jeweiligen Verarbeitungsvorrichtungen, dem Verschließen der Behältnisse und so weiter. Eine gemeinsame Weiterverarbeitung einer derartigen Anordnung, insbesondere ein Befüllen und Verschließen der pharmazeutischen Behältnisse kann beispielsweise in einer Vorrichtung, wie in WO 2011/000606 A1 beschrieben, erfolgen, indem die Anordnung der pharmazeutischen Behältnisse durch eine Trägerplatte fixiert den einzelnen Verfahrensschritten unterzogen wird.

Derartige Trägerplatten umfassen einen, eine Mehrzahl von Durchgangsöffnungen zur Aufnahme der pharmazeutischen Behältnisse aufweisenden Aufnahmebereich, einen eine Grundebene definierenden Handhabungsbereich zum Angriff an der Trägerplatte und/oder Lagerung dieser, eine unter Belastung als Druckseite fungierende Oberseite und eine als Zugseite fungierende Unterseite sowie Mittel zur Erhöhung der Steifigkeit der Trägerplatte. Die Trägerplatte ist in der Regel aus Kunststoff gefertigt. Die Trägerplatte mit den in diesen positionierten und zentrierten pharmazeutischen Behältnissen ist bei den vorgenannten Verarbeitungs- und Transportprozessen jedoch einer Vielzahl von Belastungen ausgesetzt, die zu unerwünschten Verformungen an dieser führen und das Handling der Trägerplatte sowie die Qualität der Verarbeitungsprozesse erheblich beeinträchtigen. Im Einzelnen sind irreversible Kaltverformungen an der Trägerplatte durch die automatischen an dieser zur Realisierung der genannten Verarbeitungsprozesse vorzunehmenden Manipulationsvorgänge zu beobachten. Ein weiterer wesentlicher Nachteil besteht darin, dass eine sichere manuelle Handhabung pharmazeutische Behältnisse enthaltender Trägerplatten durch deren geringe Eigensteifigkeit nicht gegeben ist. Insbesondere können aufgrund der sich unter Belastung einstellenden Durchbiegung und der damit verbundenen Lageabweichungen der pharmazeutischen Behältnisse untereinander und gegenüber den Weiterverarbeitungsvorrichtungen beispielsweise die Füllhöhe bei Befüllung der Behältnisse und/oder die Stopfensetzhöhe beim Verschließen variieren. Verzüge aufgrund undefinierter Lagerbedingungen sowie Lufteinschlüsse beim Stopfensetzen sind ebenfalls nicht auszuschließen. Die Fehlerquote ist dadurch relativ hoch und führt entweder zur Nichtverwendbarkeit der davon betroffenen pharmazeutischen Behältnisse oder bei gewünschter Vermeidung zu einem unverhältnismäßig hohen Steuerungsaufwand und/oder dem Vorsehen von Zusatzmaßnahmen bei der Handhabung.

Aus der US 2009/100802A1, der EP 2 119 436 A1, der WO 2011/007194 A1 sowie der US 3 643 812 A sind unterschiedliche Transport- und/oder Lagereinrichtungen für pharmazeutische Behältnisse, insbesondere Spritzen, Fläschchen und Karpulen bekanntgeworden, bei denen eine Vielzahl der pharmazeutischen Behältnisse in einer definierten Position lagerbar sind.

Der Erfindung liegt die Aufgabe zugrunde, eine Trägerplatte für pharmazeutische Behältnisse der eingangs genannten Art derart weiterzuentwickeln, dass die genannten Nachteile vermieden werden, die manuelle als auch automatisierte Handhabung der vorkonfektionierten Anordnung deutlich verbessert wird und die Fehlerquote gesenkt.

Die erfindungsgemäße Lösung ist durch die Merkmale der Ansprüche 1 und 11 charakterisiert. Vorteilhafte Ausgestaltungen sind in den Unteransprüchen wiedergegeben.

Die Trägerplatte für pharmazeutische Behältnisse, insbesondere Spritzen, Fläschchen und Karpulen, umfassend einen, eine Mehrzahl von Durchgangsöffnungen zur Aufnahme der pharmazeutischen Behältnisse aufweisenden Aufnahmebereich, einen eine Grundebene definierenden Handhabungsbereich zur Handhabung der Trägerplatte, insbesondere zum Angriff an der Trägerplatte zu Transport- und Verarbeitungszwecken und/oder Lagerung dieser, eine unter Belastung als Druckseite fungierende Oberseite und eine als Zugseite fungierende Unterseite sowie Mittel zur Erhöhung der Steifigkeit der Trägerplatte, ist erfindungsgemäß dadurch gekennzeichnet, dass die Mittel zur Erhöhung der Steifigkeit zumindest einen Kastenprofilbereich zwischen zumindest zwei Durchgangsöffnungen auf der Oberseite der Trägerplatte umfassen.

Der Begriff Durchgangsöffnung ist funktional zu verstehen. Darunter werden alle Durchgänge beschreibenden geometrischen Ausbildungen verstanden, welche geeignet sind, die pharmazeutischen Behältnisse im Sinne einer Durchführung aufzunehmen und je nach Ausführung diese durch Kraft- und/oder Formschluss, beispielsweise Klemmung oder Einhängen in einer vordefinierten Lage in beziehungsweise an der Trägerplatte zu halten. Die Durchgangsöffnung kann dabei je nach Erfordernis mit konstantem Querschnitt oder veränderbarem Querschnitt in Durchführungsrichtung ausgeführt sein. Die Festlegung der Lage der einzelnen Durchgangsöffnung sowie die Anordnung der einzelnen Durchgangsöffnungen zueinander, die geometrische Form, insbesondere die Wahl der Querschnittsgeometrie und/oder des Querschnittsprofils in Führungsrichtung sowie die Auslegung der einzelnen Durchgangsöffnung erfolgen als Funktion der gewünschten Anordnung, insbesondere Packungsdichte und geometrischen Ausgestaltung und Auslegung der aufzunehmenden pharmazeutischen Behältnisse.

Unter Druckseite wird die Seite der Trägerplatte verstanden, die bei Aufnahme der pharmazeutischen Behältnisse die durch das Gewicht dieser bedingte Last aufnimmt und auf Biegung beansprucht wird. Die Zugseite entspricht der entgegengesetzt zu dieser ausgerichteten Seite der Trägerplatte.

Das erfindungsgemäße Vorsehen zumindest eines Kastenprofilbereiches erhöht die Biege- und Verwindungssteifigkeit in diesem Anordnungsbereich erheblich und gewährleistet dadurch eine sichere Lagefixierung der pharmazeutischen Behältnisse in diesem Bereich zueinander und gegenüber der Trägerplatte.

Der zumindest eine Kastenprofilbereich ist dazu zur Zugseite der Trägerplatte einseitig offen ausgeführt. Zur Ausbildung des die Trägerplatte versteifenden Kastenprofils ist die dieser gegenüberliegende mittlere, das Kastenprofil beschreibende Fläche gegenüber der Grundebene im wesentlichen, vorzugsweise parallel und erhöht angeordnet und über Stege bildende Flächenbereiche mit der Grundebene gekoppelt. Neben der Erhöhung der Stabilität der Trägerplatte durch Erhöhung der Biege- und Torsionssteifigkeit kann das Kastenprofil kostengünstig mit nur geringerem Materialmehrverbrauch, wenn überhaupt hergestellt werden. Die maximalen Außenabmessungen der Trägerplatte in der Horizontalebene bleiben davon unberührt.

Bezüglich der Ausbildung, Anordnung und Auslegung des einzelnen Kastenprofilbereiches besteht eine Vielzahl von Möglichkeiten. Zur Gewährleistung einer hohen Stabilität ist der zumindest ein Kastenprofilbereich jedoch immer zumindest im Bereich der theoretisch maximalen Durchbiegung der Trägerplatte angeordnet. Die Biegelinie der bestückten Trägerplatte kann dadurch erheblich abgeflacht werden.

Die Mittel zur Erhöhung der Steifigkeit können gemäß einer ersten Ausbildung eine Vielzahl von zur Zugseite der Trägerplatte einseitig offenen und miteinander über in der Grundebene oder versetzt zu dieser angeordnete Verbindungsbereiche gekoppelten Kastenprofilbereichen zwischen wenigstens zwei oder mehreren zueinander benachbart angeordneten Durchgangsöffnungen umfassen. In diesem Fall wird die Steifigkeit über eine Vielzahl einzelner Kastenprofilmodule und deren Anordnung erzielt. Eine derartige Anordnung kann bei erforderlichem Vorsehen weiterer Funktionselemente sinnvoll sein.

In einer besonders vorteilhaften zweiten Ausbildung umfassen die Mittel zur Erhöhung der Steifigkeit nur einen zur Zugseite der Trägerplatte einseitig offenen, einen Kastenmodul bildenden Kastenprofilbereich zwischen allen Durchgangsöffnungen. Dieser beschreibt eine gitterartige Struktur, die zwischen den einzelnen Durchgangsöffnungen erstreckend angeordnet ist. Diese Lösung bietet den Vorteil einer einfachen Herstellung bei gleichzeitig sehr hoher erzielbarer Biege- und Verwindungssteifigkeit der Trägerplatte über den gesamten Aufnahmebereich für pharmazeutische Behältnisse.

Zur Erhöhung der Funktionskonzentration bildet in einer Weiterentwicklung jeweils ein einzelner zur Zugseite der Trägerplatte einseitig offener Kastenprofilbereich zumindest auch einen Teilbereich einer Wandung zumindest einer Durchgangsöffnung mit aus. Diese Lösung ist insbesondere bei einteiliger Ausführung der gesamten Trägerplatte als Spritzgussteil besonders einfach und materialsparend realisierbar.

In einer weiteren Weiterentwicklung umfassen die Mittel zur Erhöhung der Steifigkeit auf der Zugseite der Trägerplatte angeordnete und jeweils zwei einander benachbarte Durchgangsöffnungen miteinander verbindende Rippen zur weiteren Erhöhung der Steifigkeit. Diese Rippen sind in besonders vorteilhafter Ausgestaltung im Bereich der Grundebene, vorzugsweise innerhalb eines einzelnen Kastenprofilbereiches angeordnet. Dadurch wird auch eine in Höhenrichtung gegenüber dem Stand der Technik kaum vergrößerte Ausführung der Trägerplatte realisiert.

Bezüglich der Materialwahl der Trägerplatte bestehen grundsätzlich keine Restriktionen. In vorteilhafter Weise werden im Hinblick auf den Herstellungsprozess komplexerer Geometrien und die Eigenschaften jedoch Kunststoffe verwendet. Hierbei finden beispielsweise Polyprophylen oder Polyester Verwendung.

In einer besonders vorteilhaften und zeit- und kostengünstig herstellbaren Ausbildung ist die Trägerplatte mit all ihren Bestandteilen als integrales Bauteil, insbesondere Spritzgussteil ausgeführt. Dies bietet den Vorteil, dass der Kastenprofilbereich und die weiteren Mittel zur Erhöhung der Steifigkeit auch bei komplexeren Geometrien auf einfache Art und Weise und präzise fertigbar sind.

Ist die Trägerplatte in einer alternativen Ausführung mehrteilig ausgebildet, besteht die Möglichkeit der Nachrüstung von Mitteln zur Erhöhung der Steifigkeit bei herkömmlichen Trägerplatten durch Anbindung der Kastenprofilbereiche mittels Kraft- und/oder Form- und/oder Stoffschluss an die Durchgangsöffnungen und die jeweilige Grundebene.

Um ein Verdrehen von in die Durchgangsöffnung der Trägerplatte eingesetzten pharmazeutischen Behältern zu verhindern, kann vorgesehen sein, dass die Trägerplatte mit einer Einrichtung zur Verdrehsicherung der einzelnen, in die Durchgangsöffnung eingesetzten pharmazeutischen Behälter versehen ist. Die Einrichtung umfasst bevorzugt in einer Ausführungsform sich kreuzende Stege, die auf der Oberseite der Trägerplatte, insbesondere im Bereich des Kastenprofils zwischen nebeneinander liegenden Durchgangsöffnungen angeordnet ist. Obwohl die beschriebene Ausführungsform kreuzende Stege für die Einrichtung der Verdrehsicherung beschreibt, ist eine derartige Ausgestaltung nicht zwingend. Es sind für den Fachmann jedwede Ausgestaltungen denkbar, die z. B. einen Anschlag für einen Spritzenkragen aufweisen können, um so ein Verdrehen einer in eine Durchgangsöffnung eingesetzten Spritze zu verhindern.

Eine erfindungsgemäße Trägerplatte ist in besonders vorteilhafter Ausführung in einer Transport- und/oder Lagereinrichtung für pharmazeutische Behältnisse, insbesondere Spritzen, Fläschchen und Karpulen einsetzbar. Diese umfasst einen Aufnahmebehälter, in welchem die Trägerplatte im un- als auch bestückten Zustand in ihrer Lage definiert lagerbar ist und zumindest eine Abdeckung des Aufnahmebehälters. Weitere Funktionselemente, wie beispielsweise Dichteinrichtungen sind vorsehbar. Durch die hohe Eigensteifigkeit der Trägerplatte bleiben die in diesen gelagerten Behältnissen hinsichtlich ihrer Lage zueinander beim Transport und der Lagerung auch über einen längeren Zeitraum in der Durchgangsöffnung zentriert und definiert. Beschädigungen der Behältnisse können somit vermieden werden.

Die erfindungsgemäße Lösung wird nachfolgend anhand von Figuren erläutert. Darin ist im Einzelnen folgendes dargestellt:
- Figur 1: zeigt eine besonders vorteilhafte erste Ausführung einer Trägerplatte in Perspektivansicht in einer Ansicht auf die Oberseite;
- Figur 2: zeigt die Trägerplatte gemäß Figur 1 in Perspektivansicht in einer Ansicht auf die Unterseite;
- Figur 3: zeigt eine Ansicht A-A gemäß Figur 1;
- Figur 4: zeigt die erfindungsgemäße Trägerplatte beim Einsatz in einer Aufnahmeeinrichtung zu Transportzwecken in Explosionsdarstellung;
- Figur 5: zeigt eine besonders vorteilhafte zweite Ausführung einer Trägerplatte in einer Ansicht auf die Oberseite mit einer Verdrehsichereinrichtung;
- Figur 6: zeigt einen Ausschnitt der Trägerplatte gemäß Figur 5 in einer geschnittenen Ansicht mit Kastenprofilbereich und Verdrehsichereinrichtungen entlang Schnitt A-A;
- Figur 7: zeigt eine Draufsicht auf eine zweite Ausführungsform einer Trägerplatte gemäß Figur 5 mit eingesetzten pharmazeutischen Behältnissen, insbesondere Spritzen;
- Figur 8: zeigt eine dreidimensionale Ansicht einer zweiten Ausführungsform einer Trägerplatte gemäß Figur 5 mit eingesetzten pharmazeutischen Behältnissen, insbesondere Spritzen.

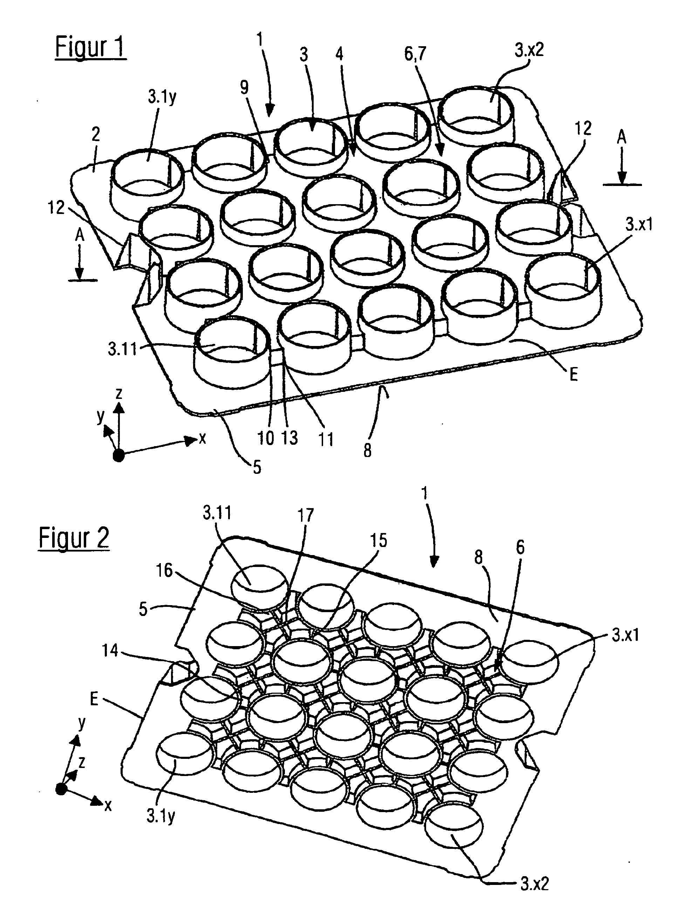

Figur 1 verdeutlicht in perspektivischer Darstellung eine besonders vorteilhafte erste Ausbildung einer erfindungsgemäß ausgeführten Trägerplatte 1 für hier nicht dargestellte pharmazeutische Behältnisse, insbesondere Spritzenkörper, Fläschchen und Karpulen in einer Ansicht auf die bei Aufnahme der pharmazeutischen Behältnisse als Druckseite fungierenden Oberseite 2. Die Trägerplatte 1 ist im dargestellten Fall rechteckförmig ausgeführt. Diese Grundform ermöglicht eine optimale Anordnung der Behältnisse sowie eine günstige Handhabung. Andere Grundformen sind jedoch in Abhängigkeit des Einsatzfalles ebenfalls denkbar. Zur Verdeutlichung der einzelnen Richtungen ist beispielhaft ein Koordinatensystem an die Trägerplatte 1 angelegt. Die X-Richtung verdeutlicht die Längsrichtung, die Y-Richtung die Breitenrichtung und die Z-Richtung die Höhenrichtung.

Die Trägerplatte 1 ist in dieser besonders vorteilhaften ersten Ausbildung als integrales Bauteil, d.h. einteilig ausgeführt. Vorzugsweise erfolgt die Fertigung als Spritzgussteil. Die Trägerplatte 1 umfasst einen, eine Mehrzahl von Durchgangsöffnungen 3 zur Aufnahme der pharmazeutischen Behältnisse aufweisenden Aufnahmebereich 4. Die Durchgangsöffnungen sind hier in vorteilhafter Weise gleichmäßig in Zeilen und Spalten angeordnet und mit 3.11 bis 3.x1, 3.1y bis 3.xy mit x>1, y>1 bezeichnet. Andere Anordnungen, regelmäßig oder unregelmäßig sind ebenfalls denkbar. Die Trägerplatte 1 umfasst ferner einen, eine Grundebene E definierenden Handhabungsbereich 5 zum Handling der Trägerplatte 1 sowie Mittel 6 zur Erhöhung der Steifigkeit der Trägerplatte 1. Die Mittel 6 zur Erhöhung der Steifigkeit umfassen erfindungsgemäß zumindest einen auf der Oberseite 2 angeordneten Kastenprofilbereich 7 zwischen zumindest zwei Durchgangsöffnungen 3. Der Kastenprofilbereich 7 verbindet dabei diese zumindest zwei Durchgangsöffnungen 3. Der zumindest eine Kastenprofilbereich 7 ist zur als Zugseite der Trägerplatte 1 fungierenden Unterseite 8 einseitig offen ausgeführt und wird im Wesentlichen durch drei, ein U- oder C-Profil beschreibende Seitenflächen und die das Profil verschließenden Flächen begrenzt. Der Profilquerschnitt ist durch zwei Seitenflächen und eine dazwischen angeordnete mittlere Fläche 9 charakterisiert, welche vorzugsweise parallel zur Grundebene E und in Höhenrichtung versetzt zu dieser angeordnet ist. Die mittlere Fläche 9 liegt der offenen Seite des Kastenprofilbereiches 7 gegenüber und kann eben ausgeführt sein. Möglich wäre auch ein diagonaler Verlauf oder ein Verlaud, der sich wie eine Bogenbrücke über das Areal der Kastenform erstreckt. Die geometrische Form bestimmt sich in Abhängigkeit der Anordnung und Erstreckung eines einzelnen Kastenprofilbereiches 7. Die Kopplung der mittleren Fläche 9 mit der Grundebene E erfolgt über die Stege bildenden Seitenflächen 10, 11, deren Formgebung sich ebenfalls in Abhängigkeit der Anordnung und Erstreckung eines einzelnen Kastenprofilbereiches 7 bestimmt.

Die Anordnung des zumindest einen Kastenprofilbereiches 7 erfolgt zumindest im Bereich der theoretisch maximalen Durchbiegung der Trägerplatte 1. In Figur 1 ist eine besonders vorteilhafte erste Ausbildung eines Kastenprofilbereiches 7 wiedergegeben, welcher sich vorzugsweise über den gesamten Aufnahmebereich 4 erstreckt. Der Kastenprofilbereich 7 bildet hier einen Kastenmodul, der einteilig mit dem Handhabungsbereich 5 ausgeführt ist und in Funktionskonzentration Wandungen der Durchgangsöffnungen 3.11 bis 3.xy mit ausbildet.

Die Trägerplatte 1 ist im dargestellten Fall einteilig als Spritzgussteil ausgeführt, wobei die Durchgangsöffnungen 3.11 bis 3.xy vorzugsweise bei der Herstellung dieser mit ausgeformt werden. Die Durchgangsöffnungen 3.11 bis 3.xy sind hier durch zylindrische geformte Elemente charakterisiert, die mit dem Kastenmodul als bauliche Einheit ausgeführt sind, wobei ein Teilbereich der Umfangsfläche eines derartig zylindrisch geformten Elementes die Funktion der als Stege fungierende Seitenflächen 10, 11 mit übernimmt. Der in Höhenrichtung erfolgende Verschluss des Kastenprofilbereiches 7 erfolgt über Stege bildende Flächen 13.

Der Kastenmodul ist derart ausgeführt, dass der durch diesen gebildete Kastenprofilbereich 7 alle inneren Durchgangsöffnungen vollständig und die in den jeweils äußeren Spalten und Zeilen angeordneten Durchgangsöffnungen 3.11 bis 3.x1, 3.11 bis 3.1y, 3.1y bis 3.xy und 3.x1 bis 3.xy zumindest teilweise in Umfangsrichtung umschließt, wobei der Umschlingungswinkel für die in den äußeren Zeilen und Spalten angeordneten Durchgangsöffnungen vorzugsweise gleich oder größer 180° beträgt. Der Kastenprofilbereich 7 wird in diesem Bereich über die Stege bildenden Flächen 13 zwischen jeweils zwei benachbarten Durchgangsöffnungen in den jeweils äußeren Spalten und Zeilen verschlossen.

Der Handhabungsbereich 5 kann in Abhängigkeit der Anforderungen an die Funktion der Trägerplatte 1 ausgeführt sein. Im dargestellten Fall ist dieser durch beidseitig angeordnete Einrichtungen 12 charakterisiert, welche beispielsweise der Aufnahme der Trägerplatte 1 in einem Aufnahmebehälter zu Transportzwecken und der Ausbildung von Angriffsflächen zum Transport dienen.

Die Figur 2 zeigt in perspektivischer Darstellung die in Figur 1 dargestellte Trägerplatte 1 in einer Ansicht von unten auf die Unterseite 8. In besonders vorteilhafter Weise umfassen die Mittel 6 zur Erhöhung der Steifigkeit auf der Zugseite der Trägerplatte 1 angeordnete Rippen 14, 15, 16 und 17. Die Rippen 14 verbinden dabei die einzelnen Durchgangsöffnung bildenden Wandungen der einander benachbarten Durchgangsöffnungen innerhalb einer Reihe x. Die Rippen 15 dienen der Verbindung der einzelnen Durchgangsöffnungen bildenden Wandungen einander benachbarter Durchgangsöffnungen in einer Spalte y. Die Rippen 16 und 17 dienen der Verbindung einer Durchgangsöffnung mit den weiteren unmittelbar benachbarten Durchgangsöffnungen. Die Rippen 16, 17 sind vorzugsweise sich einander kreuzend angeordnet. Die Anordnung der Rippen 14 bis 17 erfolgt vorzugsweise im Bereich der Grundebene E und daher in der dargestellten Ausführung im Bereich des offenen Endes des Kastenprofilbereiches 7. Gemäß einer besonders vorteilhaften Ausbildung sind die Rippen 14 bis 17 vorzugsweise vollständig innerhalb der Erstreckung des Kastenprofilbereiches 7 in Höhenrichtung angeordnet und dadurch in diesem integriert. Die Rippen 14 bis 17 verbinden dabei jeweils Durchgangsöffnungen im Bereich ihres Außenumfanges miteinander, wobei sich die Rippen 14 bis 17 vorzugsweise bis zur Erstreckung der Wandbereiche der Durchgangsöffnungen in Richtung zur Zugseite der Trägerplatte 1 erstrecken.

Wie bereits ausgeführt, ist die Trägerplatte 1 vorzugsweise als Kunststoffbauteil und Hinsichtlich der Herstellung als Spritzgussteil ausgeführt. Die Herstellung durch Spritzgießen erlaubt sehr komplexe Versteifungsgeometrien mit einfachen Mitteln in nur einem Prozessschritt.

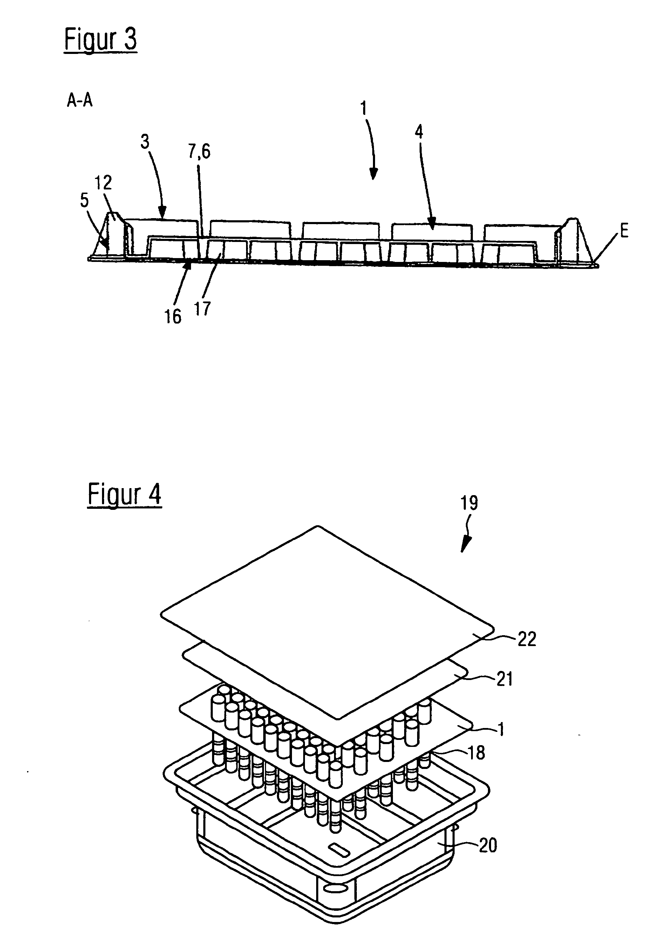

Die Figur 3 zeigt die Trägerplatte 1 in einer Schnittansicht A-A gemäß Figur 1. Ersichtlich sind der Handhabungsbereich 5, die Einrichtungen 12 sowie die Durchgangsöffnungen 3 und das diese verbindende Kastenmodul als Kastenprofilbereich 7. Ferner in dieser Schnittansicht ersichtlich sind die, die Außenumfänge der Durchgangsöffnungen 3 verbindenden Rippen 16, 17.

Figur 4 verdeutlicht die erfindungsgemäße erste Ausführungsform einer Trägerplatte 1 mit von dieser aufgenommenen pharmazeutischen Behältnissen 18 in einer Transport- und Lagereinrichtung 19 in Explosionsdarstellung, umfassend einen wannenförmigen Aufnahmebehälter 20. In den Aufnahmebehälter 20 kann die mit den pharmazeutischen Behältnissen 18 bestückte Trägerplatte 1 eingebracht und beispielsweise mittels der Einrichtungen 12 und/oder über weitere mögliche, die Lage der Trägerplatte 1 im wannenförmigen Aufnahmebehälter 20 fixierende Einrichtungen eingehangen beziehungsweise gelagert werden. Im dargestellten Fall sind eine Abdeckplatte 21 und eine Dichtplatte 22 zum Verschließen des Aufnahmebehälters 20 vorgesehen, die nach Lagerung der bestückten Trägerplatte 1 den Aufnahmebehälter 20 verschließend über der Trägerplatte 1 angeordnet werden.

Die Lagerung der Trägerplatte1 im Aufnahmebehälter kann durch Kraft- und/oder Formschluss realisiert werden. Die konkrete Auswahl erfolgt in Abhängigkeit des Einsatzfalles. Der Verschluss wird in der Regel durch eine Abdeckung ermöglicht, die mit weiteren Funktionseinheiten, wie Dichteinrichtungen, Dämmungsmaterialien e.t.c. kombiniert werden kann.

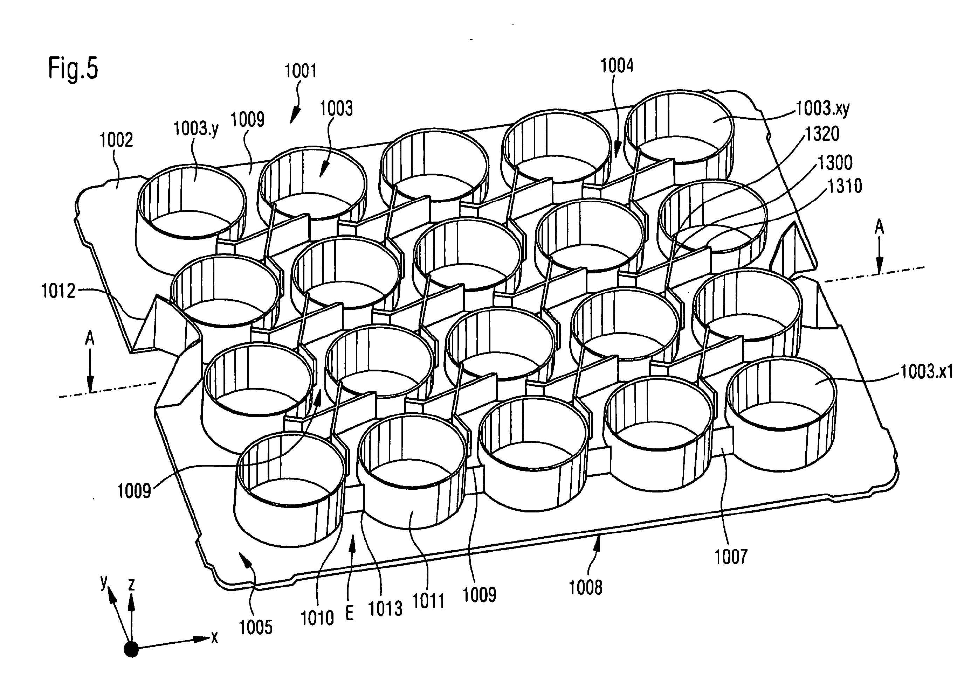

In den Figuren 5 bis 8 ist eine zweite Ausführungsform einer erfindungsgemäßen Trägerplatte gezeigt. Gleiche Bauteile wie in den Figuren1 bis 4 sind mit um 1000 erhöhten Bezugsziffern bezeichnet.

Figur 5 verdeutlicht in perspektivischer Darstellung eine besonders vorteilhafte zweite Ausbildung einer erfindungsgemäß ausgeführten Trägerplatte 1001 für hier nicht dargestellte pharmazeutische Behältnisse, insbesondere Spritzenkörper, Fläschchen oder Karpulen in einer Ansicht auf die bei Aufnahme der pharmazeutischen Behältnisse als Druckseite fungierenden Oberseite 1002. Die Trägerplatte 1001 ist im dargestellten Fall rechteckförmig ausgeführt. Diese Grundform ermöglicht eine optimale Anordnung der Behältnisse sowie eine günstige Handhabung. Andere Grundformen sind jedoch in Abhängigkeit des Einsatzfalles ebenfalls denkbar. Zur Verdeutlichung der einzelnen Richtungen ist beispielhaft ein Koordinatensystem an die Trägerplatte 1001 angelegt. Die X-Richtung verdeutlicht die Längsrichtung, die Y-Richtung, die Breitenrichtung und die Z-Richtung die Höhenrichtung.

Die Trägerplatte 1001 ist in dieser besonders vorteilhaften zweiten Ausbildung als integrales Bauteil, d.h. einteilig ausgeführt. Vorzugsweise erfolgt die Fertigung als Spritzgussteil. Die Trägerplatte 1001 umfasst einen, eine Mehrzahl von Durchgangsöffnungen 1003 zur Aufnahme der pharmazeutischen Behältnisse aufweisenden Aufnahmebereich 1004. Die Durchgangsöffnungen sind hier in vorteilhafter Weise gleichmäßig in Zeilen und Spalten angeordnet und mit 1003.11 bis 1003.x1, 1003.1y bis 1003.xy mit x>1, y>1 bezeichnet. Andere Anordnungen, regelmäßig oder unregelmäßig sind ebenfalls denkbar. Die Trägerplatte 1001 umfasst ferner einen, eine Grundebene E definierenden Handhabungsbereich 1005 zum Handling der Trägerplatte 1001 sowie Mittel 1006 zur Erhöhung der Steifigkeit der Trägerplatte 1001. Die Mittel 1006 zur Erhöhung der Steifigkeit umfassen erfindungsgemäß zumindest einen auf der Oberseite 1002 angeordneten Kastenprofilbereich 1007 zwischen zumindest zwei Durchgangsöffnungen 1003. Der Kastenprofilbereich 1007 verbindet dabei diese zumindest zwei Durchgangsöffnungen 1003. Der zumindest eine Kastenprofilbereich 7 ist zur als Zugseite der Trägerplatte 1001 fungierenden Unterseite 1008 einseitig offen ausgeführt und wird im Wesentlichen durch drei, ein U- oder C-Profil beschreibende Seitenflächen und die das Profil verschließende Flächen begrenzt. Der Profilquerschnitt ist durch zwei Seitenflächen und eine dazwischen angeordnete mittlere Fläche 1009 charakterisiert, welche vorzugsweise parallel zur Grundebene E und in Höhenrichtung versetzt zu dieser angeordnet ist. Die mittlere Fläche 1009 liegt der offenen Seite des Kastenprofilbereiches 7 gegenüber und ist vorzugsweise eben ausgeführt. Die geometrische Form bestimmt sich in Abhängigkeit der Anordnung und Erstreckung eines einzelnen Kastenprofilbereiches 1007. Die Kopplung der mittleren Fläche 1009 mit der Grundebene E erfolgt über die Stege bildenden Seitenflächen 1010, 1011, deren Formgebung sich ebenfalls in Abhängigkeit der Anordnung und Erstreckung eines einzelnen Kastenprofilbereiches 1007 bestimmt.

Die Anordnung des zumindest einen Kastenprofilbereiches 1007 erfolgt zumindest im Bereich der theoretisch maximalen Durchbiegung der Trägerplatte 1001. In Figur 1 ist eine besonders vorteilhafte erste Ausbildung eines Kastenprofilbereiches 1007 wiedergegeben, welcher sich vorzugsweise über den gesamten Aufnahmebereich 1004 erstreckt. Der Kastenprofilbereich 7 bildet hier einen Kastenmodul, der einteilig mit dem Handhabungsbereich 1005 ausgeführt ist und in Funktionskonzentration Wandungen der Durchgangsöffnungen 1003.11 bis 1003.xy mit ausbildet.

Die Trägerplatte 1001 ist im dargestellten Fall einteilig als Spritzgussteil ausgeführt, wobei die Durchgangsöffnungen 1003.11 bis 1003.xy vorzugsweise bei der Herstellung dieser mit ausgeformt werden. Die Durchgangsöffnungen 1003.11 bis 1003.xy sind hier durch zylindrische geformte Elemente charakterisiert, die mit dem Kastenmodul als bauliche Einheit ausgeführt sind, wobei ein Teilbereich der Umfangsfläche eines derartig zylindrisch geformten Elementes die Funktion der als Stege fungierende Seitenflächen 1010, 1011 mit übernimmt. Der in Höhenrichtung erfolgende Verschluss des Kastenprofilbereiches 1007 erfolgt über Stege bildende Flächen 1013.

Der Kastenmodul ist derart ausgeführt, dass der durch diesen gebildete Kastenprofilbereich 1007 alle inneren Durchgangsöffnungen vollständig und die in den jeweils äußeren Spalten und Zeilen angeordneten Durchgangsöffnungen 1003.11 bis 1003.x1, 1003.11 bis 1003.1y, 1003.1y bis 1003.xy und 1003.x1 bis 1003.xy zumindest teilweise in Umfangsrichtung umschließt, wobei der Umschlingungswinkel für die in den äußeren Zeilen und Spalten angeordneten Durchgangsöffnungen vorzugsweise gleich oder größer 180° beträgt. Der Kastenprofilbereich 1007 wird in diesem Bereich über die Stege bildenden Flächen 1013 zwischen jeweils zwei benachbarten Durchgangsöffnungen in den jeweils äußeren Spalten und Zeilen verschlossen.

Der Handhabungsbereich 1005 kann in Abhängigkeit der Anforderungen an die Funktion der Trägerplatte 1001 ausgeführt sein. Im dargestellten Fall ist dieser durch beidseitig angeordnete Einrichtungen 1012 charakterisiert, welche beispielsweise der Aufnahme der Trägerplatte 1001 in einem Aufnahmebehälter zu Transportzwecken und der Ausbildung von Angriffsflächen zum Transport dienen.

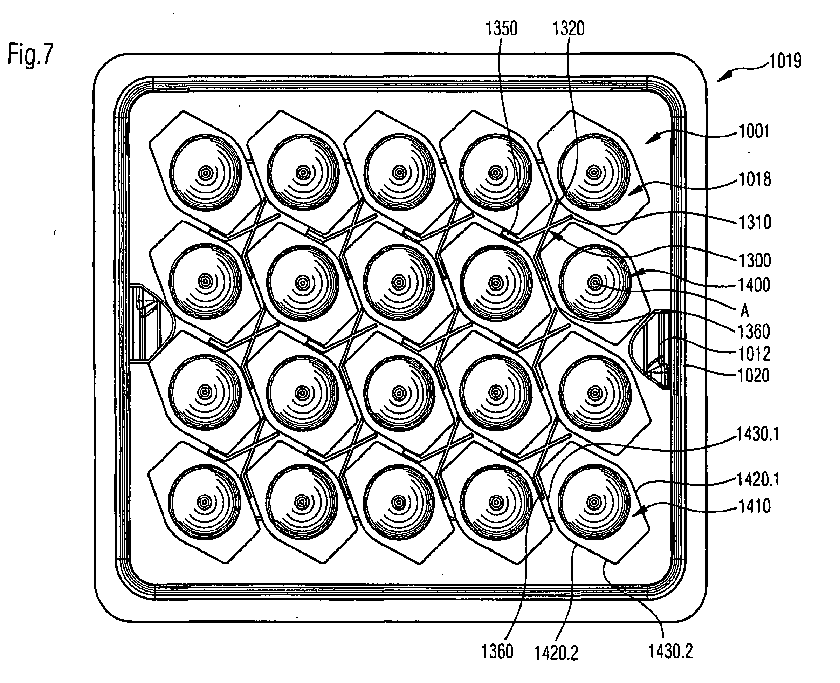

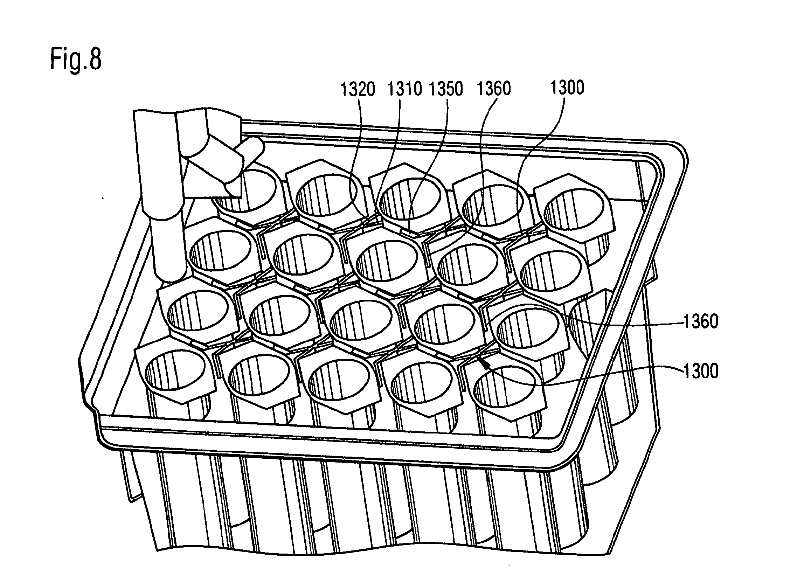

Zusätzlich zur ersten Ausgestaltung in den Figuren 1 bis 4 weist die zweite Ausführungsform der Erfindung zwischen benachbarter Durchgangsöffnung 1003 eine Verdrehsicherungseinrichtung 1300 auf. In vorliegender Ausführungsform ist die Verdrehsicherungseinrichtung 1300 aus zwei Stegen 1310, 1320 gebildet, die gekreuzt sind. Die Stege 1310, 1320 der Verdrehsicherungseinrichtung 1300 sind auf der mittleren Fläche 1009 im Kastenprofilbereich 1007 angeordnet. Die Stege 1310, 1320 der Verdrehsicherung übernehmen zusätzlich zu den Rippen 1014, 1015, 1016, 1017 an der Unterseite 1008 der Trägerplatte 1001 eine Versteifungsfunktion. Darüber hinaus sind die Stege 1310, 1320 so ausgebildet und angeordnet, dass sie eine Verdrehsicherung der in die Durchgangsöffnungen 1003 eingesetzten pharmazeutischen Behältnisse, insbesondere verfüllbare Spritzen, Fläschchen oder Karpulen übernimmt. So kann nicht nur eine vorgegebene Position der pharmazeutischen Behältnisse zueinander eingehalten werden, wie bei der ersten Ausführungsform, sondern auch die Winkellage der in die bevorzugt rotationssymmetrische Durchgangsöffnung 1003 eingesetzten pharmazeutischen Behältnisse. Detailliert ist dies für Spritzen in den Figuren 7 und 8 gezeigt. Die Stege 1310, 1320 sind exakt an die in die Durchgangsöffnung 1003 eingesetzten pharmazeutischen Behältnisse angepasst. Die Verdrehsicherung dient insbesondere dazu, ein Verdrehen der pharmazeutischen Behältnisse im Handhabungsbereich 1005, insbesondere im Bereich der Einrichtungen 1012 zu vermeiden. Ganz wichtig ist dies, wenn in die Einrichtung 1012 kein Fingereingriff, z. B. zur manuellen Bearbeitung, zur Verfügung steht, sondern ein maschineller

Eingriff, bei der z. B. Zentrierstifte in die Einrichtung 1012 eingreifen. Würden sich bei einer derartig maschinell prozessierten Einheit z. B. eine Spritze wegen fehlender Verdrehsicherung verdrehen bzw. aus der vorgegebenen Winkellage rotieren, so kann der Bereich der Einrichtung 1012 überdeckt werden. Ein maschineller Eingriff ist dann nicht mehr möglich und die Prozesssicherheit wird beeinträchtigt.

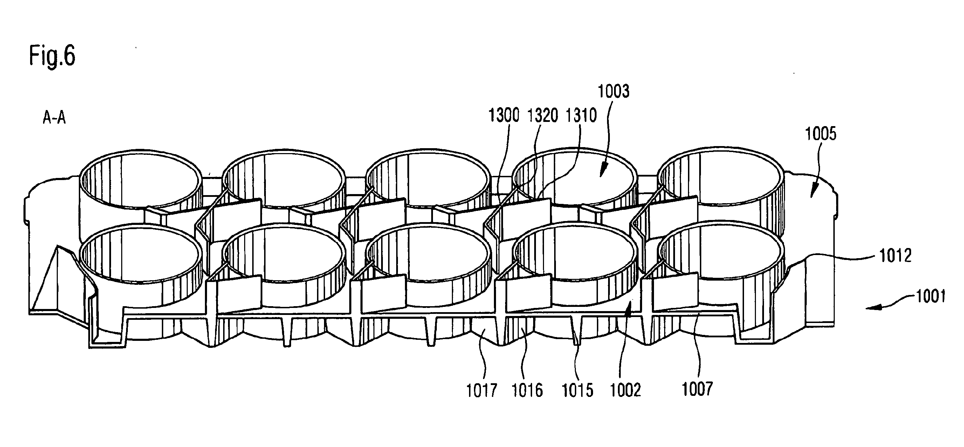

Die Figur 6 zeigt in perspektivischer Darstellung einen Ausschnitt der in Figur 5 dargestellten Trägerplatte 1001 in einer Schnittansicht von oben. Im Schnitt A-A in Figur 5 ist deutlich zu erkennen, dass in besonders vorteilhafter Weise die Mittel 1006 zur Erhöhung der Steifigkeit auf der Zugseite der Trägerplatte 1001 angeordnete Rippen 1014, 1015, 1016 und 1017 umfassen. Die Rippen 1014 (nicht gezeigt) analog zu Rippe 14 in Figur 2 verbinden dabei die einzelnen Durchgangsöffnung bildenden Wandungen der einander benachbarten Durchgangsöffnungen innerhalb einer Reihe x. Die Rippen 1015 dienen der Verbindung der einzelnen Durchgangsöffnungen 1003 bildenden Wandungen einander benachbarter Durchgangsöffnungen in einer Spalte y. Die Rippen 1016 und 1017 dienen der Verbindung einer Durchgangsöffnung 1003 mit den weiteren unmittelbar benachbarten Durchgangsöffnungen 1003. Die Rippen 1016, 1017 sind vorzugsweise sich einander kreuzend angeordnet. Die Anordnung der Rippen 1014 bis 1017 erfolgt vorzugsweise im Bereich der Grundebene E und daher in der dargestellten Ausführung im Bereich des offenen Endes des Kastenprofilbereiches 1007. Gemäß einer besonders vorteilhaften Ausbildung sind die Rippen 1014 bis 1017 vorzugsweise vollständig innerhalb der Erstreckung des Kastenprofilbereiches 1007 in Höhenrichtung angeordnet und dadurch in diesem integriert. Die Rippen 1014 bis 1017 verbinden dabei jeweils Durchgangsöffnungen im Bereich ihres Außenumfanges miteinander, wobei sich die Rippen 1014 bis 1017 vorzugsweise bis zur Erstreckung der Wandbereiche der Durchgangsöffnungen in Richtung zur Zugseite der Trägerplatte 1001 erstrecken.

Wie bereits ausgeführt, ist die Trägerplatte 1001 vorzugsweise als Kunststoffbauteil und Hinsichtlich der Herstellung als Spritzgussteil ausgeführt. Die Herstellung durch Spritzgießen erlaubt sehr komplexe Versteifungsgeometrien mit einfachen Mitteln in nur einem Prozessschritt.

Figur 7 zeigt eine zweite Ausführungsform der Trägerplatte 1001 mit von dieser in den Durchgangsöffnungen 1003 aufgenommenen pharmazeutischen Behältnissen 1018 in einer Transport- und Lagereinrichtung 1019 in einer Draufsicht, umfassend einen wannenförmigen Aufnahmebehälter 1020, wie er detailliert in Figur 8 gezeigt wird. In den Aufnahmebehälter 1020 kann die mit den pharmazeutischen Behältnissen 1018, hier Spritzenkörper 1400, bestückte Trägerplatte 1001 eingebracht und beispielsweise mittels der Einrichtungen 1012 und/oder über weitere mögliche, die Lage der Trägerplatte 1001 im wannenförmigen Aufnahmebehälter 1020 fixierende Einrichtungen eingehangen beziehungsweise gelagert werden. Zum Verschließen des Aufnahmebehälters 1020 kann eine Abdeckplatte und eine Dichtplatte vorgesehen sein, die nach Lagerung der bestückten Trägerplatte 1001 verschließend über der Trägerplatte 1001 angeordnet werden.

Die Lagerung der Trägerplatte1001 im Aufnahmebehälter kann durch Kraft- und/oder Formschluss realisiert werden. Die konkrete Auswahl erfolgt in Abhängigkeit des Einsatzfalles. Der Verschluss wird in der Regel durch eine Abdeckung ermöglicht, die mit weiteren Funktionseinheiten, wie Dichteinrichtungen, Dämmungsmaterialien etc. kombiniert werden kann.

Deutlich zu erkennen sind in Figur 7 die Einrichtungen 1300 zur Verdrehsicherung, umfassend die Stege 1310, 1320. Die einzelnen Spritzenkörper umfassen einen Spritzenkragen 1410 mit je zwei Seitenbereichen 1420.1, 1420.2, umfassend je zwei Seitenbereichen 1430.1, 1430.2. Die Stege 1310, 1320 weisen je einen Anschlagsabschnitt 1350, 1360 auf, die an einem Seitenbereich 1430.1, 1430.2 des Spritzenkragens anliegen. Durch das Anliegen wenigstens eines Anschlagsabschnittes 1350, 1360 an wenigstens einem Seitenbereich 1430.1, 1430.2 eines Spritzenkragens eines Spritzenkörpers kann verhindert werden, dass sich der Spritzenkörper um seine Achse A bzw. die Achse der Durchgangsöffnung verdreht, d.h. aus seiner Winkellage verbracht wird und so beispielsweise die Bereiche 1012 ungewollt verdeckt.

Figur 8 zeigt einen Trägerkörper 1001 der zweiten Ausführungsform, eingesetzt in einem wannenförmigen Aufnahmebehälter in einer dreidimensionalen Ansicht. Gleiche Bauteile wie in Figur 7 sind mit denselben Bezugsziffern belegt. Deutlich zu erkennen der Spritzenkörper 1400 mit Spritzenkragen 1410 und an den Spritzenkragen anschließenden zylindrischen Spritzenabschnitt 1440, der beispielsweise mit einem Pharmazeutika oder einem anderen Fluid befüllt werden kann und in eine Durchgangsöffnung 1001 eingesetzt ist.

Die erfindungsgemäße Lösung ist nicht auf die Ausführungen gemäß den Figuren beschränkt. Diese stellen lediglich eine besonders vorteilhafte Ausführung dar. Weiterentwicklungen, dieselbe Erfindung betreffend sind möglich.

### Bezugszeichenliste

- 1, 1001: Trägerplatte
- 2, 1002: Oberseite
- 3, 3.11 - 3.x1: Durchgangsöffnungen
- 3.1y - 3.xy:
- 1003, 1003.11 -:
- 1003.x1, 1003.1y -:
- 1003.xy:
- 4, 1004: Aufnahmebereich
- 5, 1005: Handhabungsbereich
- 6, 1006: Mittel zur Erhöhung der Steifigkeit
- 7, 1007: Kastenprofilbereich
- 8, 1008: Unterseite
- 9, 1009: mittlere Fläche
- 10, 1010: Seitenfläche
- 11, 1011: Seitenfläche
- 12, 1012: Einrichtung
- 13, 1013: Fläche
- 14, 1014: Rippe
- 15, 1015: Rippe
- 16, 1016: Rippe
- 17, 1017: Rippe
- 18, 1018: pharmazeutische Behältnisse
- 19, 1019: Transport- und Lagereinrichtung
- 20, 1020: Aufnahmebehälter
- 21: Abdeckplatte
- 22: Dichtplatte
- 1300: Einrichtung zur Verdrehsicherung
- 1310, 1320: Stege der Einrichtung zur Verdrehsicherung
- 1350, 1360: Anschlagabschnitte des Steges
- 1400: Spritzenkörper
- 1410: Spritzenkragen
- 1420.1, 1420.2: Langseiten
- 1430.1, 1430.2: Seitenabschnitte
- 1440: Spritzenabschnitt

## Patentansprüche

1. Trägerplatte (1) für pharmazeutische Behältnisse (18), insbesondere Spritzen, Fläschchen oder Karpulen, umfassend einen, eine Mehrzahl von Durchgangsöffnungen (3, 3.11 - 3.x1, 3.1y - 3.xy) zur Aufnahme der pharmazeutischen Behältnisse (18) aufweisenden Aufnahmebereich (4), einen eine Grundebene (E) definierenden Handhabungsbereich (5) zur Handhabung der Trägerplatte (1), insbesondere zum Angriff an der Trägerplatte (1) und/oder Lagerung dieser, eine unter Belastung als Druckseite fungierende Oberseite (2) und eine als Zugseite fungierende Unterseite (8) sowie Mittel (6) zur Erhöhung der Steifigkeit der Trägerplatte (1),
**dadurch gekennzeichnet,**
**dass** die Mittel (6) zur Erhöhung der Steifigkeit zumindest einen Kastenprofilbereich (7) zwischen zumindest zwei Durchgangsöffnungen (3, 3.11 - 3.x1, 3.1y - 3.xy) an der Oberseite (2) der Trägerplatte (1) umfassen.

2. Trägerplatte (1) nach Anspruch 1,
**dadurch gekennzeichnet,**
**dass** der zumindest eine Kastenprofilbereich (7) zur Zugseite der Trägerplatte (1) einseitig offen ausgeführt ist, die dieser gegenüberliegende mittlere, das Kastenprofil beschreibende Fläche (9) parallel zur und gegenüber der Grundebene (E) erhöht angeordnet und über Stege bildende Flächenbereiche (10, 11, 13) mit der Grundebene (E) gekoppelt ist.

3. Trägerplatte (1) nach Anspruch 1 oder 2,
**dadurch gekennzeichnet,**
**dass** der zumindest eine Kastenprofilbereich (7) zumindest im Bereich der theoretisch maximalen Durchbiegung der Trägerplatte (1) angeordnet ist.

4. Trägerplatte (1) nach einem der Ansprüche 1 bis 3,
**dadurch gekennzeichnet,**
**dass** die Mittel (6) zur Erhöhung der Steifigkeit eine Vielzahl von zur Zugseite der Trägerplatte (1) einseitig offenen und miteinander über in der Grundebene (E) oder versetzt zu dieser angeordnete Verbindungsbereiche gekoppelten Kastenprofilbereichen (7) zwischen wenigstens zwei oder mehreren zueinander benachbart angeordneten Durchgangsöffnungen (3, 3.11 - 3.x1, 3.1 y - 3.xy) umfassen.

5. Trägerplatte (1) nach einem der Ansprüche 1 bis 3,
**dadurch gekennzeichnet,**
**dass** die Mittel (6) zur Erhöhung der Steifigkeit einen zur Zugseite der Trägerplatte (1) einseitig offenen, einen Kastenmodul bildenden Kastenprofilbereich (7) zwischen allen Durchgangsöffnungen (3, 3.11 - 3.x1, 3.1y - 3.xy) umfassen.

6. Trägerplatte (1) nach einem der Ansprüche 1 bis 5,
**dadurch gekennzeichnet,**
**dass** der jeweils einzelne zur Zugseite der Trägerplatte (1) einseitig offene Kastenprofilbereich (7) zumindest einen Teilbereich einer Wandung zumindest einer Durchgangsöffnung (3, 3.11 - 3.x1, 3.1y - 3.xy) bildet.

7. Trägerplatte (1) nach einem der Ansprüche 1 bis 6,
**dadurch gekennzeichnet,**
**dass** die Mittel (6) zur Erhöhung der Steifigkeit auf der Zugseite der Trägerplatte (1) angeordnete und jeweils zwei einander benachbarte Durchgangsöffnungen (3, 3.11 - 3.x1, 3.1y - 3.xy) miteinander verbindende Rippen (14, 15, 16, 17) umfasst.

8. Trägerplatte (1) nach Anspruch 7,
**dadurch gekennzeichnet,**
**dass** die Rippen (14, 15, 16, 17) innerhalb der vertikalen Erstreckung eines einzelnen Kastenprofilbereiches (7) angeordnet sind.

9. Trägerplatte (1) nach einem der Ansprüche 1 bis 8,
**dadurch gekennzeichnet,**
**dass** die Trägerplatte (1) als integrales Bauteil, insbesondere Spritzgussteil ausgeführt ist.

10. Trägerplatte (1) nach einem der Ansprüche 1 bis 9,
**dadurch gekennzeichnet,**
**dass** der Aufnahmebereich (4) und der Handhabungsbereich (5) der Trägerplatte (1) als integrales Bauteil, insbesondere Spritzgussteil ausgeführt ist und der einzelne Kastenprofilbereich (7) kraft- und/oder formschlüssig und/oder stoffschlüssig mit diesem verbindbar ist.

11. Trägerplatte nach einem der Ansprüche 1 bis 10,
**dadurch gekennzeichnet, dass**
die Trägerplatte Einrichtungen (1300) zur Verdrehsicherung umfasst.

12. Trägerplatte nach Anspruch 11,
**dadurch gekennzeichnet, dass**
die Einrichtung (1300) zur Verdrehsicherung Stege (1310, 1320) umfasst.

13. Trägerplatte nach Anspruch 12,
**dadurch gekennzeichnet, dass**
die Stege (1310, 1320) wenigstens einen Anschlagabschnitt (1350, 1360), insbesondere für einen Spritzenkragen (1410), umfasst.

14. Transport- und/oder Lagereinrichtung (19) für pharmazeutische Behältnisse (18), insbesondere Spritzen, Fläschchen und Karpulen, umfassend eine Trägerplatte (1) gemäß einem der Ansprüche 1 bis 13, die in einem Aufnahmebehälter (20) in ihrer Lage definiert lagerbar ist und zumindest eine, den Aufnahmebehälter (20) verschließende Abdeckung (21).

## Claims

1. A carrier plate (1) for pharmaceutical containers (18), especially syringes, small bottles or ampoules, comprising a receiving region (4) having a plurality of through-openings (3, 3.11 - 3.x1, 3.1 y - 3.xy) for accommodating the pharmaceutical containers (18), a handling region (5) that defines a base plane (E) for handling the carrier plate (1), in particular for engaging on the carrier plate (1) and/or for bearing the same, an upper side (2) acting as a pressure side under load and a bottom side (8) acting as a tension side, and means (6) for increasing the stiffness of the carrier plate (1), **characterized in that** the means (6) for increasing the stiffness comprise at least box section region (7) between at least two through-openings (3, 3.11 - 3.x1, 3.1y - 3.xy) on the upper side (2) of the carrier plate (1).

2. A carrier plate (1) according to claim 1, **characterized in that** the at least one box section region (7) is arranged to be open on one side towards the tension side of the carrier plate (1), the middle area (9) which is opposite thereof and describes the box section is arranged in an elevated manner parallel to and opposite of the base plane (E), and is coupled to the base plane (E) via surface areas (10, 11, 13) which form webs.

3. A carrier plate (1) according to claim 1 or 2, **characterized in that** the at least one box section region (7) is arranged at least in the region of the theoretically maximum deflection of the carrier plate (1).

4. A carrier plate (1) according to one of the claims 1 to 3, **characterized in that** the means (6) for increasing the stiffness comprise a plurality of box section regions (7) between at least two or more mutually adjacently arranged through-openings (3, 3.11 - 3.x1, 3.1y- 3.xy), said box section regions being open on one side towards the tension side of the carrier plate (1) and being coupled to each other via connecting regions arranged in the base plane (E) or offset thereto.

5. A carrier plate (1) according to one of the claims 1 to 3, **characterized in that** the means (6) for increasing the stiffness comprise a box section region (7) between all through-openings (3, 3.11 - 3.x1, 3.1y- 3.xy), said box section region being open on one side towards the carrier plate (1) and forming a box module.

6. A carrier plate (1) according to one of the claims 1 to 5, **characterized in that** the respective individual box section region (7) which is open on one side towards the tension side of the carrier plate (1) forms at least a partial region of a wall of at least one through-opening (3, 3.11 - 3.x1, 3.1 y - 3.xy).

7. A carrier plate (1) according to one of the claims 1 to 6, **characterized in that** the means (6) for increasing the stiffness comprise ribs (14, 15, 16, 17) which are arranged on the tension side of the carrier plate (1) and respectively connect two mutually adjacent through-openings (3, 3.11 - 3.x1, 3.1y - 3.xy) to each other.

8. A carrier plate (1) according to claim 7, **characterized in that** the ribs (14, 15, 16, 17) are arranged within the vertical extension of an individual box section region (7).

9. A carrier plate (1) according to one of the claims 1 to 8, **characterized in that** the carrier plate (1) is arranged as an integral component, especially an injection-moulded component.

10. A carrier plate (1) according to one of the claims 1 to 9, **characterized in that** the receiving region (4) and the handling region (5) of the carrier plate (1) are arranged as an integral component, especially an injection-moulded component, and the individual box section region (7) is connectable to said component in a frictionally engaged and/or interlocking and/or materially bonded manner.

11. A carrier plate according to one of the claims 1 to 10, **characterized in that** the carrier plate comprises devices (1300) for anti-twist locking.

12. A carrier plate according to claim 11, **characterized in that** the device (1300) for anti-twist locking comprises webs (1310, 1320).

13. A carrier plate according to claim 12, **characterized in that** the webs (1310, 1320) comprise at least one stop section (1350, 1360), especially for a syringe collar (1410).

14. A transport and/or storage device (19) for pharmaceutical containers (18), especially syringes, small bottles and ampoules, comprising a carrier plate (1) according to one of the claims 1 to 13, which can be stored in a receiving container (20) in a defined manner with respect to its position, and at least one cover (21) closing the receiving container (20).

## Revendications

1. Plaque-support (1) pour des récipients pharmaceutiques (18), en particulier des seringues, des flacons ou des cartouches, comprenant une zone de réceptacles (4) qui présente une pluralité d'ouvertures de passage (3, 3.11 - 3.x1, 3.1y - 3.xy) destinées à recevoir les récipients pharmaceutiques (18), une zone de manipulation (5) définissant un plan de base (E) pour la manipulation de la plaque-support (1), en particulier pour saisir la plaque-support (1) et/ou poser celle-ci, une face supérieure (2) servant de côté de poussée sous contrainte et une face inférieure (8) servant de côté de traction ainsi que des moyens (6) pour accroître la rigidité de la plaque-support (1),
**caractérisée en ce que** les moyens (6) pour accroître la rigidité présentent au moins une zone de profilé en caisson (7) entre au moins deux ouvertures de passage (3, 3.11 - 3.x1, 3.1y - 3.xy) sur le face supérieure (2) de la plaque-support (1).

2. Plaque-support (1) selon la revendication 1, **caractérisée en ce que** l'au moins une zone de profilé en caisson (7) est ouverte d'un côté vers le côté de traction de la plaque-support (1) et la surface (9) centrale lui faisant face et décrivant le profilé en caisson est disposée parallèlement au plan de base (E) et surélevée par rapport à celui-ci et couplée au plan de base (E) par des zones de surface (10, 11, 13) formant des barrettes.

3. Plaque-support (1) selon la revendication 1 ou 2, **caractérisée en ce que** l'au moins une zone de profilé en caisson (7) est disposée au moins dans la zone de fléchissement maximal théorique de la plaque-support (1).

4. Plaque-support (1) selon l'une des revendications 1 à 3, **caractérisée en ce que** les moyens (6) pour accroître la rigidité comprennent une pluralité de zones de profilé en caisson (7) ouvertes d'un côté vers le côté de traction de la plaque-support (1) et couplées entre elles par des zones de liaison disposées dans le plan de base (E) ou décalées par rapport à celui-ci entre au moins deux ou plusieurs ouvertures de passage (3, 3.11 - 3.x1, 3.1y - 3.xy) disposées au voisinage les unes des autres.

5. Plaque-support (1) selon l'une des revendications 1 à 3, **caractérisée en ce que** les moyens (6) pour accroître la rigidité comprennent une zone de profilé en caisson (7) ouverte d'un côté vers le côté de traction de la plaque-support (1) et formant un module de caisson entre toutes les ouvertures de passage (3, 3.11 - 3.x1, 3.1y - 3.xy).

6. Plaque-support (1) selon l'une des revendications 1 à 5, **caractérisée en ce que** chaque zone de profilé en caisson (7) ouverte d'un côté vers le côté de traction de la plaque-support (1) forme au moins une partie d'une paroi d'au moins une ouverture de passage (3, 3.11 - 3.x1, 3.1y - 3.xy).

7. Plaque-support (1) selon l'une des revendications 1 à 6, **caractérisée en ce que** les moyens (6) pour accroître la rigidité comprennent des nervures (14, 15, 16, 17) disposées sur le côté de traction de la plaque-support (1) et reliant deux par deux des ouvertures de passage (3, 3.11 - 3.x1, 3.1y - 3.xy) voisines.

8. Plaque-support (1) selon la revendication 7, **caractérisée en ce que** les nervures (14, 15, 16, 17) sont disposées à l'intérieur de l'étendue verticale de chaque zone de profilé en caisson (7).

9. Plaque-support (1) selon l'une des revendications 1 à 8, **caractérisée en ce que** la plaque-support (1) est réalisée comme une pièce d'un seul tenant, en particulier une pièce moulée par injection.

10. Plaque-support (1) selon l'une des revendications 1 à 9, **caractérisée en ce que** la zone de réceptacles (4) et la zone de manipulation (5) de la plaque-support (1) sont conformées comme une pièce d'un seul tenant, en particulier une pièce moulée par injection, et chaque zone de profilé en caisson (7) peut être reliée à celle-ci par adhérence et/ou correspondance de forme et/ou solidarité de matière.

11. Plaque-support selon l'une des revendications 1 à 10, **caractérisée en ce qu'**elle comprend des dispositifs (1300) pour empêcher sa torsion.

12. Plaque-support selon la revendication 11, **caractérisée en ce que** le dispositif (1300) pour empêcher la torsion comprend des barrettes (1310, 1320).

13. Plaque-support selon la revendication 12, **caractérisée en ce que** les barrettes (1310, 1320) comprennent au moins une partie de butée (1350, 1360), en particulier pour un collet de seringue (1410).

14. Dispositif de transport et/ou de stockage (19) des récipients pharmaceutiques (18), en particulier des seringues, des flacons ou des cartouches, comprenant une plaque-support (1) selon l'une des revendications 1 à 13 qui peut être supportée dans un contenant (20) dans une position définie et au moins un couvercle (21) qui ferme le contenant (20).
